# EUROPEAN PATENT SPECIFICATION

(11) **EP 1 523 946 B1**
(45) Date of publication and mention of the grant of the patent: **17.12.2008**
(21) Application number: 03741426.5
(22) Date of filing: 16.07.2003
(51) Int. Cl.: A61B 17/128

(54) **CLIP OPERATION DEVICE**
CLIP-OPERATIONSGERÄT
DISPOSITIF DE MANIPULATION D'UNE PINCE

(30) Priority: 19.07.2002 JP 2002211371
(43) Date of publication of application: 20.04.2005
(73) Proprietor: Olympus Corporation, Tokyo 151-0072 (JP)
(72) Inventor: OKADA, Tsutomu, Tachikawa-shi, Tokyo 190-0012 (JP)
(74) Representative: von Hellfeld, Axel
(86) International application number: PCT/JP2003/009048
(87) International publication number: WO 2004/008975

(56) References cited:
- WO-A-01/49186
- WO-A-99/20183
- WO-A1-99/20183
- JP-A- 8 280 701
- JP-Y2- 4 026 091
- US-A- 5 766 189
- US-A- 5 868 663
- US-A1- 2002 045 909

## Description

### Technical Field

The present invention relates to a clip manipulating device or a clip device for an organic tissue in a cavity of a living body or the like as an object of treatment.

### Background Art

Clip devices that are used in combination with an endoscope to arrest bleeding from a region in the body cavity are described in Jpn. UM Appln. KOKOKU Publication No. 4-26091 and Jpn. Pat. Appln. KOKOKU Publication No. 63-6016, now published as JP 60103946 A.

In the device described in Jpn. UM Appln. KOKOKU Publication No. 4-26091, a clip 53 is hitched by means of a coupling plate 54 to a hook 52, which is attached to the distal end of a manipulating wire 51 that is passed through a sheath 50, as shown in FIG. 23. In the device described in Jpn. Pat. Appln. KOKOKU Publication No. 63-6016 (JP60103946A), on the other hand, a hook that is attached to the distal end of a manipulating wire is hitched directly to a clip.

In either type, a rigid coupling member, such as a hook or a coupling plate, is connected to the distal end of the manipulating wire. The clip that is located at the distal end of the sheath is detachably attached to the rigid coupling member and set in place. The clip can be opened and closed or disengaged by advancing or retreating the manipulating wire.

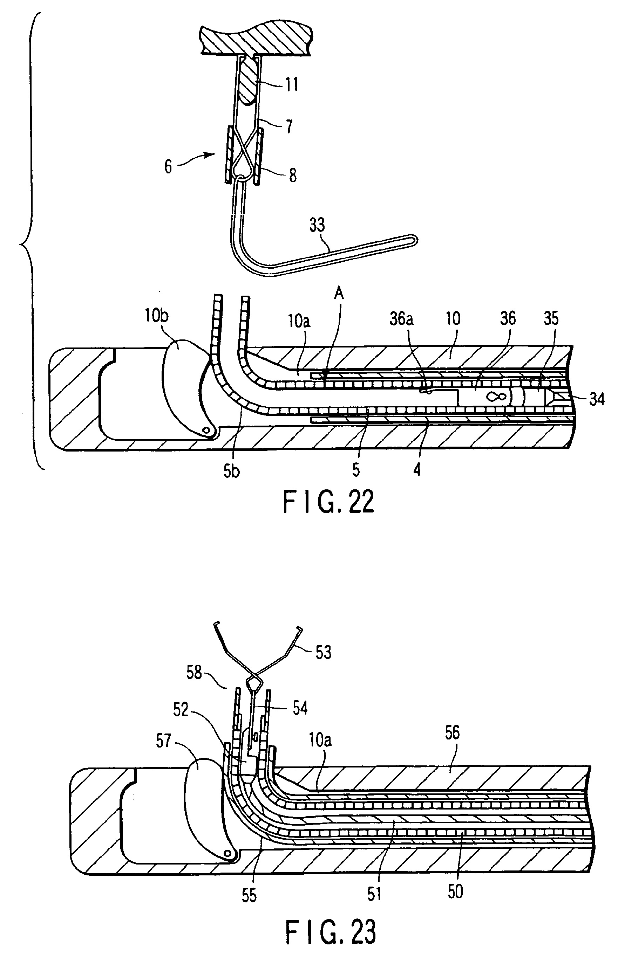

The clip device of this type is guided into the body cavity through a channel of an endoscope when it is worked. Usually, it is used in combination with a straight-viewing endoscope. Depending on the region to be treated in the body cavity, however, it sometimes is very hard to carry out treatment operation through the straight-viewing endoscope. In this case, the device is used in combination with a side-viewing endoscope, as shown in FIG. 23.

A side-viewing endoscope 56 is furnished with a forceps raising device 57 in the distal end opening of its channel. The forceps raising device 57 is used to raise a clip device 58 and guide the clip device 58 into the region to be treated in the body cavity.

Thus, in the side-viewing endoscope 56, the clip device 58 is bent and raised by means of the forceps raising device 57, so that the distal end portion of the clip device 58 is bent particularly deep.

In the clip devices described in Jpn. Pat. Appln. KOKOKU Publication No. 63-6016 (JP60103946A) and Jpn. UM Appln. KOKOKU Publication No. 4-26091, the clip is attached to the distal end of an insertion tube when it is introduced into the body cavity. Thereafter, the clip is manipulated by means of the manipulating wire, whereby the clip is disengaged from the coupling member of the manipulating wire. In the processes of operation for opening and closing the clip or disengaging the clip from the manipulating wire, the rigid coupling member, e.g., the hook or the coupling plate, on the distal end of the manipulating wire moves together with the manipulating wire in the sheath, and finally moves into the rear part of the sheath.

If the side-viewing endoscope is used, however, the distal end portion, in particular, of the sheath of the clip device is bent deep and suddenly. Therefore, the hook or the coupling plate, the rigid coupling member, is easily caught by the bent part of the sheath. It is hard for the hook or the coupling plate, the rigid coupling member, moreover, to pass smoothly through the bent part of the sheath. In some cases, therefore, a series of operations from grasping operation to disengaging operation of the clip cannot be carried out securely and smoothly.

This problem may arise when the device is used in combination with a straight- or oblique-viewing endoscope as well as when it is used in combination with the side-viewing endoscope.

Some straight- or oblique-viewing endoscopes may be furnished with a forceps raising device. Even in an endoscope that is furnished with no forceps raising device, the sheath may possibly suffer from a bent part when an insertion section itself is curved, for example. In a clip device that uses a thin sheath, in particular, the hook or the coupling plate, the rigid coupling member, is easily caught by a slightly bent part of the sheath, without regard to the type of the endoscope that is used in combination with the device. The rigid coupling member easily fails to pass smoothly through the bent part.

International Patent Application Publication No. WO 99/20183 upon which the two part form of claim 1 is based, describes a surgical clip having three or more equally spaced arms for effective engagement with body tissue regardless of clip orientation. The clip manipulating device includes a doubled manipulating wire.

United States Patent Application Publication No. 2004/0045909 describes a tissue clipping apparatus in which each clip is connected to the manipulating wire via a wire spring element.

The object of the present invention is to provide a clip manipulating device or a clip device, in which a clip (including a clip unit) located near the distal end of an insertion tube can be manipulated securely and quickly by means of manipulating means that is coupled to the clip or the clip unit.

### Disclosure of Invention

The present invention provides a clip manipulating device having the features recited in claim 1. Preferred features of the invention are recited in the dependent claims.

Thus, the present invention is a clip manipulating device, in which the distal end portion of a flexible wire, having pliability and movably passed through a flexible insertion tube capable of being inserted into a cavity of a living body, forms a junction which is detachably coupled with a clip located at the distal end portion of the insertion tube. The junction is pliable enough to follow up deformation of the insertion tube.

The present invention is a clip manipulating device, in which elongate means is passed for advance and retreat through flexible insertion means capable of being inserted into a cavity of a living body. The elongate is detachably coupled to a clip, which is located at the distal end portion of the insertion means, and is not less pliable than enough to follow up deformation of the insertion means.

The present invention is a clip manipulating device, comprising a manipulating member, which is passed for advance and retreat through a flexible insertion tube capable of being inserted into a cavity of a living body and effects grasping operation and disengaging operation of a clip located at the distal end portion of the insertion tube, and a flexible connecting member, which has one end coupled to the distal end of the manipulating member and the other end detachably coupled to the clip and is pliable enough to follow up deformation of the insertion tube.

The present invention is a clip manipulating device, in which a coupling member is provided on the distal end of a manipulating member, which is passed for movement through a flexible insertion tube capable of being inserted into a cavity of a living body, and a wire, extending from the clip and pliable enough to follow up deformation of the insertion tube, is coupled to the coupling member.

### Brief Description of Drawings

FIG. 1 is a perspective view of a clip manipulating device according to a first configuration, which does not form part of the invention ;
FIG. 2 is a view showing the distal end portion of the clip manipulating device according to the first configuration and its surroundings;
FIG. 3 is a view of the clip manipulating device according to the first configuration in a working state;
FIG. 4 is a view of the clip manipulating device according to the first configuration in a working state;
FIGS. 5A to 5D are views illustrating the action of a clip manipulating device according to a second configuration, which does not form part of the invention, in operation;
FIG. 6 is a view showing the distal end portion of a clip manipulating device according to a third configuration, which does not form part of the invention and its surroundings;
FIG. 7 is a view showing the clip manipulating device according to the third configuration in a working state;
FIGS. 8A and 8B are views illustrating the distal end portion of a clip manipulating device according to a fourth configuration, which does not form part of the invention and its surroundings;
FIGS. 9A and 9B are views illustrating the distal end portion of the clip manipulating device according to the fourth configuration and its surroundings;
FIG. 10 is a view illustrating the distal end portion of the clip manipulating device according to the fourth configuration and its surroundings;
FIG. 11 is a view illustrating the distal end portion of the clip manipulating device according to the fourth configuration and its surroundings in a working state;
FIGS. 12A and 12B are views illustrating the distal end portion of the clip manipulating device according to the fourth configuration and its surroundings in a working state;
FIG. 13 is a view illustrating the distal end portion of the clip manipulating device according to the fourth configuration and its surroundings in a working state;
FIG. 14 is a view showing the distal end portion of a clip manipulating device according to an embodiment of the invention and its surroundings;
FIG. 15 is a view showing the distal end portion of the clip manipulating device according to the embodiment of the invention and its surroundings in a working state;
FIG. 16 is a view showing the distal end portion of a clip manipulating device according to a fifth configuration, which does not form part of the invention and its surroundings;
FIG. 17 is a view showing the distal end portion of the clip manipulating device according to the fifth configuration and its surroundings in a working state;
FIG. 18 is a view showing the distal end portion of the clip manipulating device according to the fifth configuration and its surroundings in a working state;
FIG. 19 is a view showing the distal end portion of the clip manipulating device according to the fifth configuration and its surroundings;
FIG. 20 is a view showing the distal end portion of the clip manipulating device according to the fifth configuration and its surroundings in a working state;
FIG. 21 is a view showing the distal end portion of a clip manipulating device according to a sixth configuration, which does not form part of the invention and its surroundings;
FIG. 22 is a view showing the distal end portion of the clip manipulating device according to the sixth configuration and its surroundings in a working state; and
FIG. 23 is a view showing the distal end portion of a conventional clip device and its surroundings.

### (First configuration, not forming part of the invention)

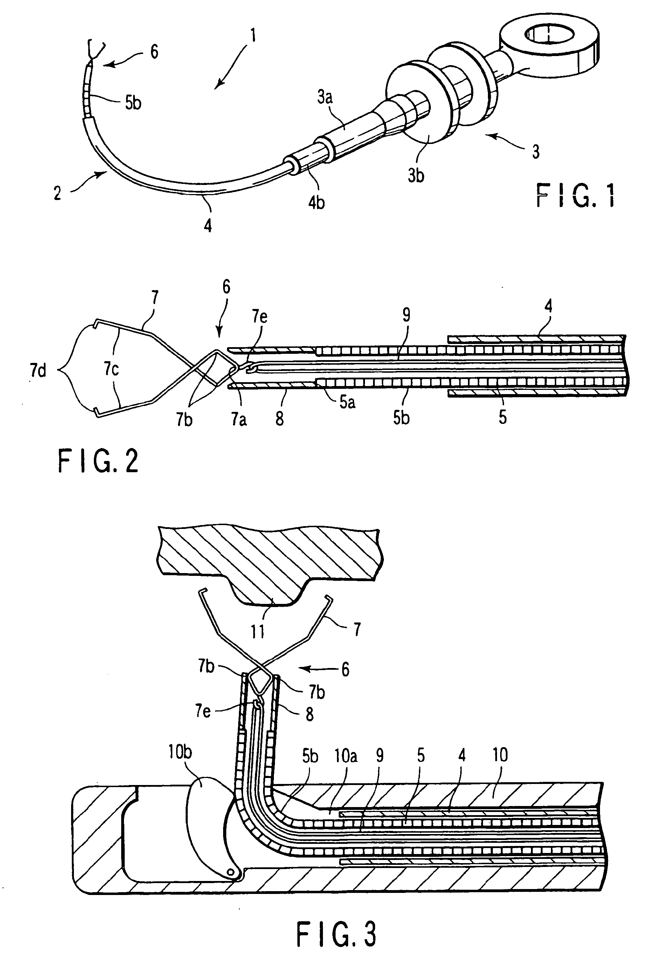

A clip manipulating device according to a first configuration, which does not form part of the invention, will be described with reference to FIGS. 1 to 4.

As shown in FIG. 1, a clip manipulating device 1 comprises an insertion section 2 and a control section 3. The insertion section 2 is formed of a flexible tube sheath 4 of, e.g., resin and a coil sheath 5 as a flexible insertion tube that is passed through the tube sheath 4. The tube sheath 4 and the coil sheath 5 are slidable relatively to each other in the longitudinal direction. The proximal end of the coil sheath 5 is connected to a body 3a of the control section 3. Further, a cylindrical stopper 4b that can abut against the distal end of the body 3a is fixedly fitted on the proximal end portion of the tube sheath 4.

As shown in FIG. 2, a clip unit 6 is attached to the distal end of the coil sheath 5. The clip unit 6 is composed of a clip 7 and a retainer pipe 8. The clip 7 is formed of a thin stainless-steel strip material that is bent in the middle. This bent part serves as a proximal end portion 7a, and those parts which are continuous with the proximal end portion 7a individually form a pair of retractable portions 7b that are spaced wider than the inside diameter of the retainer pipe 8. Strip material portions that further extend from the retractable portions 7b are crossed, and the respective one-side parts of two strip material portions that extend from the intersection for a pair of arm portions 7c, individually. Further, the respective distal end portions of the arm portions 7c are bent toward each other so that the two bent parts serve as nipping portions 7d, individually. The pair of arm portions 7c are given aptitude for spreading such that the opposed nipping portions 7d are kept wide apart, as shown in FIG. 2.

As shown in FIG. 2, a hooked anchor portion 7e, which is bent substantially in the shape of a J, is formed protruding backward from the proximal end portion of the clip 7. The anchor portion 7e constitutes a junction that is detachably coupled to the distal end of a manipulating wire 9 that is passed through the coil sheath 5. The anchor portion 7e is coupled to the distal end of the manipulating wire 9 for use as manipulating means, which will be mentioned later. When the manipulating wire 9 is hauled with a driving force of a given or higher value, the substantially J-shaped anchor portion 7e is straightened and released from the manipulating wire 9 by means of the tractive effort of the wire 9.

As shown in FIG. 2, the manipulating wire 9 is formed by doubling one flexible wire member, and two wire portions that are returned toward the handling side constitute manipulating means. A looped distal end portion that is formed by bending the one flexible wire member serves as a junction (coupling means). Thus, the looped distal end portion of the manipulating wire 9 is used as the junction (coupling means) that is anchored to the anchor portion 7e of the clip 7. The looped distal end portion of the manipulating wire 9 at which the flexible wire member is turned back is directly used to form the junction that is pliable. Since the looped extreme end portion of the manipulating wire 9 serves as a junction that is to be hitched to the anchor portion 7e, the wire member in the looped distal side part of the manipulating wire 9 forms a pliable coupling member. This junction has pliability such that the coil sheath 5 for use as the insertion tube can normally follow up possible deformation, such as flexure or bending.

The clip 7 is located near the distal end of the coil sheath 5. The proximal end portion of the clip 7 is fitted into the retainer pipe 8 that abuts against a coil sheath end 5a, and the retainer pipe 8 is loaded with a filler, such as silicone. By doing this, the clip 7 can be tacked to the coil sheath end 5a.

The proximal end side part of the manipulating wire 9 passes through the coil sheath 5 of the insertion section 2 to reach a longitudinally movable slider 3b on the control section 3, and is connected to the slider 3b. Thus, there are no rigid portions, including the junction that is anchored to the clip 7, on the rear part of the manipulating wire 9 that is situated behind the anchor portion 7e of the clip 7 and inside the coil sheath 5.

The clip unit 6 is located at the distal end of the coil sheath 5. By advancing the tube sheath 4 on the coil sheath 5, the clip unit 6 is encased in the distal end portion of the tube sheath 4. If the tube sheath 4 is retreated from this set state so that the stopper 4b abuts against the body 3a of the control section 3, as shown in FIG. 2, the respective distal end portions of the coil sheath 5 and the clip unit 6 project from the distal end of the tube sheath 4 and are exposed to the outside. When the stopper 4b runs against the body 3a of the control section 3 in this manner, the distal end portion of the coil sheath 5 forms an exposed portion 5b that projects from the distal end of the tube sheath 4.

The following is a description of the operation of the device according to the first configuration. First, with the clip unit 6 encased in the tube sheath 4, the insertion section 2 of the clip manipulating device 1 is guided into a patient's body through a channel 10a of a side-viewing endoscope 10 shown in FIG. 3.

As shown in FIG. 3, the distal end portion of the insertion section 2 of the clip manipulating device 1 is projected from the endoscope 10, the tube sheath 4 for use as a manipulating member is retreated, and the stopper 4b is caused to abut against the distal end of the body 3a of the control section 3. Thereupon, both the clip unit 6 and the exposed portion 5b of the coil sheath 5 project from the distal end of the tube sheath 4.

In this state, a forceps raising device 10b of the endoscope 10 is raised, as shown in FIG. 3. Thereupon, the exposed portion 5b of the coil sheath 5 is intensively bent and raised by means of the forceps raising device 10b, and the clip unit 6 gets into the field of the endoscope 10. The exposed portion 5b of the coil sheath 5 is long enough to be curved by means of the forceps raising device 10b. Thus, the exposed portion 5b can be securely fixed in its raised state with its middle part kept bent by means of the forceps raising device 10b.

As shown in FIG. 3, the clip unit 6 is guided to an affected region 11 in the patient's body, and operation is carried out to pull the slider 3b of the control section 3 toward the proximal end. By this operation, the manipulating wire 9 is hauled and retreated toward the proximal end. Thereupon, the retractable portions 7b of the clip 7 are drawn into and squeezed by the retainer pipe 8, and the pair of nipping portions 7d of the clip 7 spread out wide, as shown in FIG. 3.

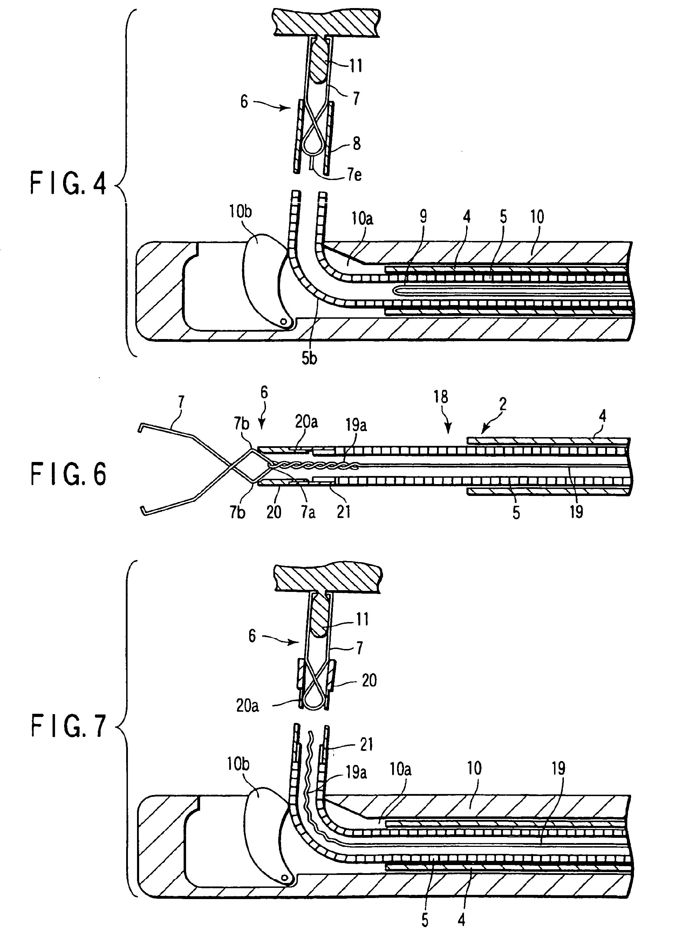

The pair of nipping portions 7d of the clip 7 in the spread state are pressed against the affected region 11, and the manipulating wire 9 is hauled and retreated by pulling the slider 3b toward the proximal end as it is. Thereupon, the respective proximal end portions of the arm portions 7c of the clip 7 are drawn into the retainer pipe 8, and the arm portions 7c of the clip 7 are closed to grasp the affected region 11, as shown in FIG. 4.

With the affected region 11 nipped by means of the clip 7 in this manner, the slider 3b is pulled toward the proximal end, and the manipulating wire 9 is hauled and retreated. Since the tractive effort to retreat the manipulating wire 9 is greater than before, the anchor portion 7e of the clip 7, having so far been engaged by the distal end of the manipulating wire 9, is deformed and elongated. Thereupon, the manipulating wire 9 is disengaged from the anchor portion 7e, as shown in FIG. 4. The clip 7 and the manipulating wire 9 are disengaged from each other, whereupon the clip unit 6 is disengaged and released from the manipulating wire 9. As shown in FIG. 4, the clip unit 6 is confined in the patient's body without ceasing to nip the affected region 11.

In a final stage for the clip unit 6 to leave the manipulating wire 9, the anchor portion 7e of the clip 7 elongates substantially straight, as shown in FIG. 4. However, the distal end of the anchor portion 7e never projects outward from the end of the retainer pipe 8. Therefore, the body wall or the like cannot be damaged by the hooked anchor portion 7e that is elongated after the disengagement.

In the present configuration, the whole manipulating wire 9 is pliable, including the distal junction formed of that part of the wire which is situated behind the anchor portion 7e of the clip 7, and includes no rigid portions. In a series of processes of clip manipulation by pulling the manipulating wire 9 toward the handling side, therefore, the wire can be smoothly moved in the coil sheath 5 without being easily caught by that part of the coil sheath 5 which is bent by means of the forceps raising device 10b. The manipulating wire 9 can be quickly advanced and retreated in the coil sheath 5 to ensure smooth manipulation. If the coil sheath 5 is deeply bent by means of the forceps raising device 10b in the side-viewing endoscope 10, in particular, the movement in the coil sheath 5 cannot be hindered. Thus, the clip manipulating device 1 can be operated securely and quickly even in the side-viewing endoscope 10.

In the present embodiment of the invention, the coupling means that is detachably coupled to the clip or the clip unit is not less pliable than enough to follow up the shape of the insertion tube. Even when the insertion tube is in a bent state, therefore, the coupling means cannot be easily caught in the middle of the interior of the insertion tube, so that it can be smoothly moved in the insertion tube.

Further, the clip or the clip unit is provided with the anchor portion that is released from the manipulating means of the manipulating wire 9 by means of the tractive effort of the given or higher value when it is hauled by the manipulating means with the tractive effort. Accordingly, rigid regions on the manipulating means side can be eliminated or reduced. Even when the insertion tube is in the bent state, therefore, the coupling means can be smoothly moved in the insertion tube without hitching with ease.

### (Second Configuration, not forming part of the invention)

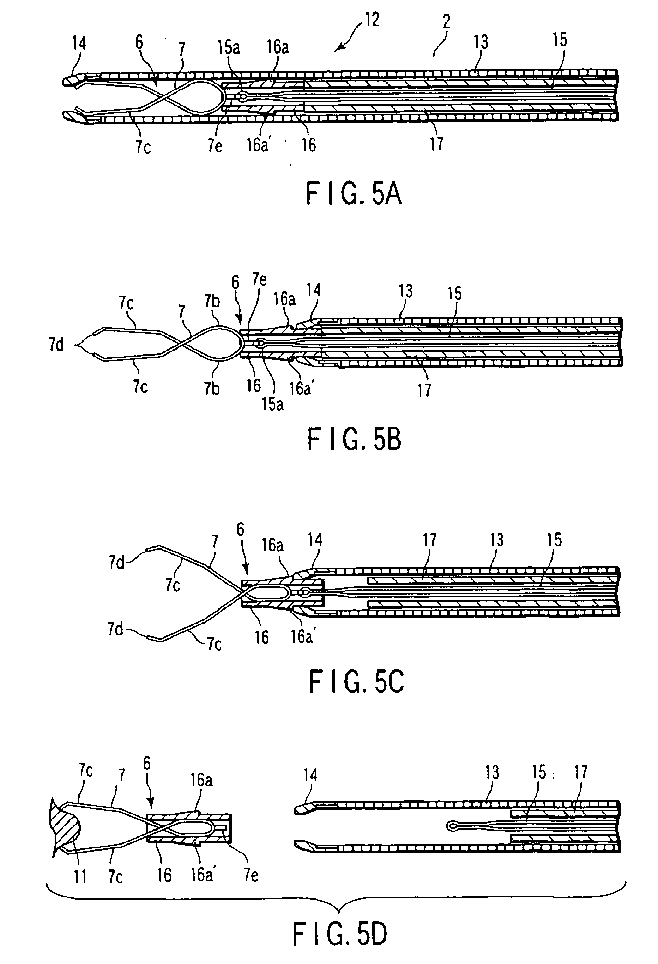

A clip manipulating device according to a second configuration, which does not form part of the invention, will be described with reference to FIGS. 5A to 5D.

A clip manipulating device 12 according to the present configuration has the following construction. In an insertion section 2 of the clip manipulating device 12, a sheath member that is equivalent to the tube sheath 4 forms a coil sheath 13. A cylindrical distal tip 14, which has a diameter smaller than the inside diameter of the coil sheath 13, is coaxially mounted and fixed on the distal end of the coil sheath 13.

A manipulating wire 15 to be coupled to a clip unit 6 is passed through a sheath-shaped push member 17 (mentioned later) in the coil sheath 13 as it is guided to the handling side through the coil sheath 13.

The manipulating wire 15 is formed by turning a pliable wire in the middle, and the turned distal side part forms a pliable junction (coupling means). An anchor portion 7e of the clip unit 6 is removably anchored to a looped portion 15a at the extreme end.

The clip unit 6 is composed of a clip 7, which is formed in the same manner as the one according to the foregoing first configuration, and a clamp ring 16 formed of resin or metal having satisfactory strength and elasticity. The clamp ring 16 has, on its outer peripheral region, a pair of or a plurality of collapsible blades 16a and 16a' as elastic projections that can be elastically deformed and project outward. The elastic projections are not limited to blade-shaped projections, and may be replaced with an elastic projecting ring or the like that encircles the clamp ring 16.

The anchor portion 7e for use as a wire connecting portion is provided on the rear end of the clip 7. The looped portion (junction) 15a of the manipulating wire 15 is coupled to the anchor portion 7e for detachable engagement. The anchor portion 7e, like the one according to the foregoing first configuration, is configured to be detachably coupled with the looped distal end portion of the manipulating wire 9 that is passed through the coil sheath 5. The anchor portion 7e is elongated to release the manipulating wire 9 for use as manipulating means (mentioned later) by means of a tractive effort of a given or higher value when the manipulating wire 9 is hauled with the tractive effort.

Further, the coil sheath 13 has therein the flexible push member 17 that surrounds the manipulating wire 15. The push member 17 is fixed to the manipulating wire 15 on the handling side.

In the present configuration, that part of the manipulating wire 15 which is also situated behind the anchor portion 7e of the clip 7 includes no rigid portions. The present configuration shares other configurations with the first configuration mentioned before.

The following is a description of the operation of the device according to the present configuration. First, the blades 16a and 16a' of the clamp ring 16 are folded, and the clip unit 6 is encased in the coil sheath 13, as shown in FIG. 5A. With the clip unit 6 encased in this manner, the insertion section 2 of the clip manipulating device 1 is introduced into the body cavity through the channel of the endoscope.

Then, the push member 17 is advanced by means of the slider 3b of the control section 3 so that the clip unit 6 is temporarily projected from the distal end of the coil sheath 13, as shown in FIG. 5B. Thereupon, the blades 16a and 16a' of the clamp ring 16, having so far been folded, expand into their original shape.

If the manipulating wire 15, along with the coil sheath 13, is pulled slightly to retreat the clamp ring 16, as shown in FIG. 5C, the blades 16a and 16a' engage the distal tip 14 and are prevented from retreating further. The coil sheath 13 is retreated leaving the distal tip 14.

The coil sheath 13, receded from the distal tip 14, is suitably raised by means of the forceps raising device of the endoscope, and the clip 7 is guided toward the affected region 11 in the patient's body. With the clip 7 pressed against the affected region 11, the slider 3b is pulled toward the handling side, and the manipulating wire 15 is hauled. Thereupon, retractable portions 7b of the clip 7 are drawn into the clamp ring 16, and the retractable portions 7b are squeezed so that the clip 7 spreads, as shown in FIG. 5C.

If the manipulating wire 15 is further retreated so that the respective proximal portions of arm portions 7c of the clip 7 are drawn into the clamp ring 16, the clip 7 can be closed to grasp the affected region 11. If the manipulating wire 15 is hauled and retreated with the affected region 11 nipped by means of the clip 7, the anchor portion 7e of the clip 7 is deformed and elongated straight so that it is disengaged from the junction at the distal end of the manipulating wire 15, as shown in FIG. 5D, whereupon the clip unit 6 leaves the insertion section 2. As shown in FIG. 5D, the clip 7 is confined in the patient's body without ceasing to nip the affected region 11.

According to the present configuration, as described above, projecting the clip unit 6 from the coil sheath 13 and manipulating the clip can be carried out by means of the slider 3b of the control section 3. Thus, the present configuration has the effect that the manipulation is easy, besides the same effects of the first configuration.

### (Third Embodiment, not forming part of the invention)

A clip manipulating device according to a third configuration, which does not form part of the invention, will be described with reference to FIGS. 6 and 7.

A clip manipulating device 18 for organic tissue according to the present configuration is constructed in the following manner. An insertion section 2 of the clip manipulating device 18 is constructed in the same manner as the one according to the first configuration mentioned before. As shown in FIG. 6, a manipulating wire 19 is passed through a coil sheath 5 of the insertion section 2. A clip unit 6 is formed of a clip 7 and a retainer pipe 20 having a small-diameter portion 20a, which will be mentioned later.

The clip 7 is constructed substantially in the same manner as the one according to the first configuration mentioned before. In the present configuration, however, it is not provided with the anchor portion 7e, and a looped portion at a proximal end portion 7a of the clip 7 is formed as an anchor portion for wire connection. The distal end wire portion of the manipulating wire 19 is passed through and anchored to the looped portion.

As shown in FIG. 6, the distal end portion of the manipulating wire 19 is formed by passing one pliable wire member through the looped portion at the proximal end portion 7a of the clip 7 and turning it backward, and then intertwining the backwardly turned wire portion with the remaining original part of the manipulating wire 19, thereby forming a stranded portion 19a for connection. The manipulating wire 19 is the weakest part of the wire member.

With this construction, the pliable coupling member portion can be formed having no rigid portions on that part of the manipulating wire 19 which is situated behind the proximal end portion 7a of the clip 7.

A cylindrical distal tip 21 is provided on the distal end of the coil sheath 5. The distal end portion of the distal tip 21 is fitted on the small-diameter portion 20a on the outer periphery of the rear end portion of the retainer pipe 20. The rear end portion of the distal tip 21 is fitted and mounted on the outer periphery of the distal end portion of the coil sheath 5. The present configuration shares other features with the first configuration mentioned before.

The device according to the present configuration differs from the aforementioned one according to the first configuration in the mode of disengagement of the clip unit 6. If the manipulating wire 19 is hauled to close the clip 7 and hauled further strongly with the affected region 11 grasped, according to the present configuration, the wire portion corresponding to the stranded portion (weak point) 19a is loosened so that the manipulating wire 19 is disengaged from the proximal end portion 7a of the clip 7. Thereupon, the clip unit 6 comes off the manipulating wire 19. The manipulating wire 19 constitutes a junction that leaves the clip 7 as the stranded portion 19a is loosened with a tractive effort of a given value not lower than enough to loosen the wire portion corresponding to the stranded portion 19a when the junction is hauled with that tractive effort. The present configuration substantially shares other configurations with the first configuration mentioned before.

According to the present configuration, the device can be used in the same manner as in the first configuration with the same functions and effects. According to the present configuration, which enjoys the same effects of the first configuration, moreover, the clip 7 is not provided with any anchor portion that extends backward, so that the retainer pipe 20 need not be lengthened to cover the backwardly extending anchor portion. The retainer pipe 20 can be shortened correspondingly. Since the distal tip 21 on the distal end of the coil sheath 5 is fitted on the retainer pipe 20, moreover, an effect can be obtained that the clip unit 6 never tilts.

### (Fourth Configuration, not forming part of the invention)

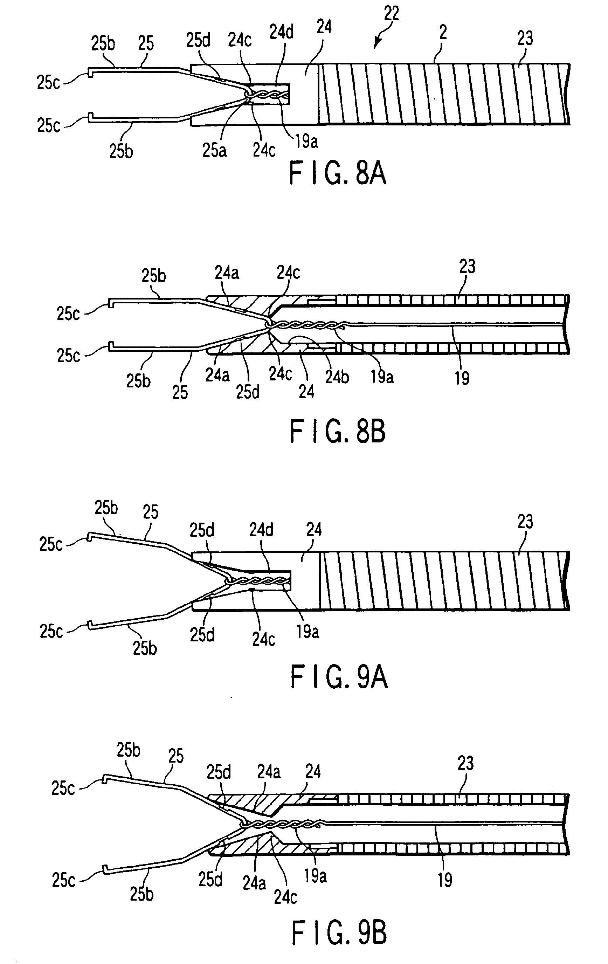

A clip manipulating device according to a fourth configuration, which does not form part of the invention, will be described with reference to FIGS. 8A to 13.

A clip manipulating device 22 for organic tissue according to the present configuration is constructed in the following manner. As shown in FIG. 8A, an insertion section 2 is formed of a coil sheath 23, and a distal tip 24 is fixedly attached to the distal end of the coil sheath 23. Defined in the distal tip 24 is a clip setting portion that detachably stores the clip and controls the clip in open-close action and plastic deformation. As shown in FIG. 8B, the inner wall portion of the distal tip 24 is formed having a pair of slope portions 24a, which are axisymmetrically located above and below so that they are more distant from each other on the distal end side. The pair of slope portions 24a range from the distal end of the distal tip 24 to the middle. As shown in FIG. 8A, a large-diameter hole 24b that is continuous with the respective rear end edges of the slope portions 24a is defined in that part which is situated on the rear end side of the rear end edges of the slope portions 24a. A pair of projections 24c are formed individually on the respective rear ends of the slope portions 24a. The projections 24c are formed by utilizing projecting end edges on the boundary between the slope portions 24a and the large-diameter hole 24b.

As shown in FIG. 8A, the distal tip 24 is provided with a slit 24d that opens in one side face thereof. A clip 25 can be attached to and detached from the slope portions 24a via the region corresponding to the distal tip 24 by utilizing the slit 24d. As shown in FIG. 9A, moreover, the slit 24d can be utilized for the recognition of the loading operation or set state of the clip 25 and the like.

As shown in FIG. 8A, the clip 25 is formed by doubling a thin stainless-steel strip material that is bent in the middle. This bent part serves as a proximal end portion 25a and arm portions 25b extend from the proximal end portion 25a. The respective distal end portions of the arm portions 25b are bent toward each other so that they serve as nipping portions 25c, individually. The proximal end portion 25a of the clip 25 is given aptitude for spreading such that the nipping portions 25c can spread wider than in the state shown in FIG. 9A. As shown in FIG. 8A, recesses 25d are formed individually in those parts of the arm portions 25b of the clip 25 which are situated near the proximal end portion 25a. The projections 24c can be fitted in and engage the recesses 25d, individually.

A manipulating wire 19, like the aforesaid one according to the third configuration has a pliable junction (coupling means) on its wire distal end side part. More specifically, the wire distal end side part of the manipulating wire 19 is passed through a looped portion of the proximal end portion 25a of the clip 25 and doubled. The turned wire portion is led backward and intertwined and joined with the remaining original wire portion, whereupon a stranded portion 19a is formed. Also in the present configuration, therefore, there are no rigid portions on that part of the manipulating wire 19 which is situated behind the proximal end portion 25a of the clip 25.

The coil sheath 23 and the manipulating wire 19 are connected to the body 3a of the control section 3 and the slider 3b, respectively.

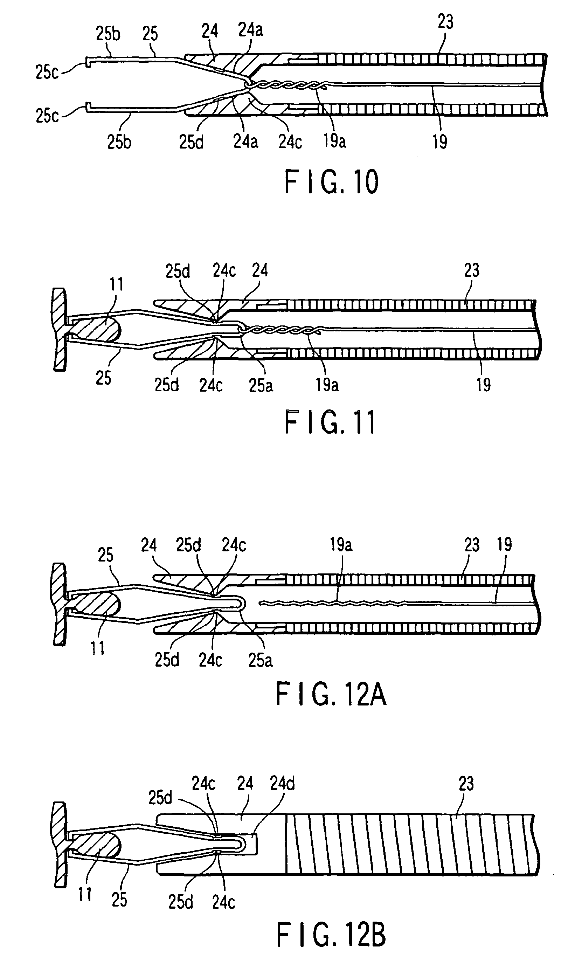

The following is a description of the operation of the device according to the present configuration. First, the respective proximal end side parts of the arm portions 25b of the clip 25 are encased in the distal tip 24, as shown in FIGS. 8A and 8B. In this state, the insertion section 2 of the clip manipulating device 12 is guided into the body cavity through the channel of the endoscope. The slider 3b of the control section 3 is advanced a little way, and the manipulating wire 19 is loosened to advance the clip 25 for a short distance. Thereupon, the arm portions 25b are spread by the aptitude of the clip 25 for spreading, as shown in FIGS. 9A and 9B. If the slider 3b is pulled toward the handling side, in contrast with this, the arm portions 25b of the clip 25 get into the distal tip 24, and the respective outer surfaces of the arm portions 25b come individually into contact with the slope portions 24a. Thereupon, the arm portions 25b of the clip 25 are closed against the aptitude for spreading (see FIGS. 10 and 11).

Thereupon, the forceps raising device of the endoscope is manipulated so that the clip 25 is guided into the affected region 11 in the body cavity, as shown in FIG. 11. Then, the manipulating wire 19 is advanced to spread and press the clip 25 against the affected region 11. The manipulating wire 19 is pulled by means of the slider with the clip 25 pressed against the affected region 11. Thereupon, the clip 25 is closed to grasp the affected region 11, as shown in FIG. 11. As this is done, the proximal end portion 25a of the clip 25 is plastically deformed by the projections 24c of the distal tip 24. After the projections 24c are cleared, the projections 24c are fitted individually into the recesses 25d of the clip 25, thereby anchoring the clip 25.

If the manipulating wire 19 is further retreated in this anchored state, the stranded portion 19a loosens, as shown in FIG. 12. The manipulating wire 19 is disengaged from the proximal end portion 25a of the clip 25.

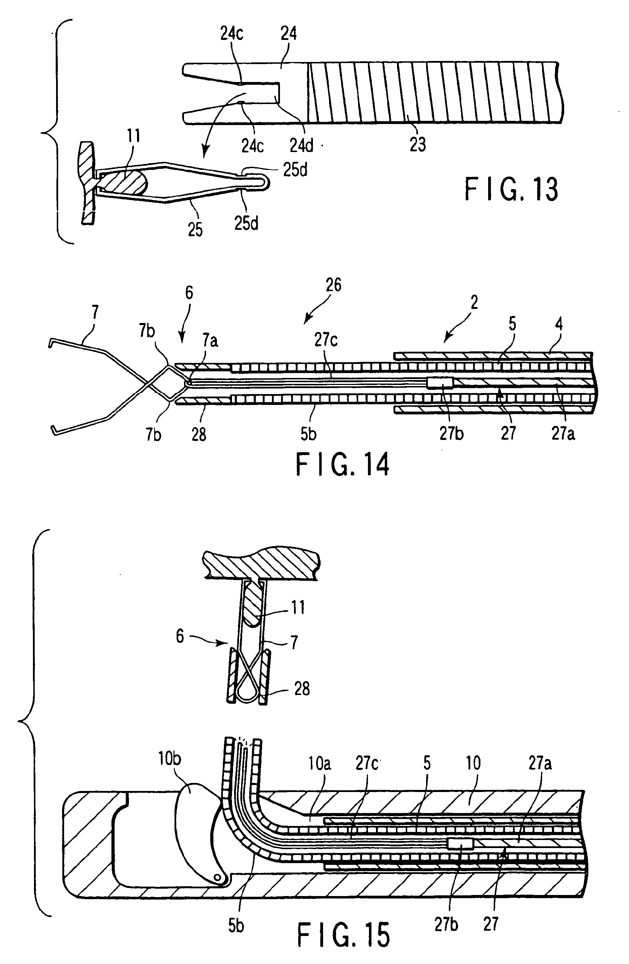

The clip 25 is allowed to be disengaged sideways from the slit 24d of the distal tip 24. As shown in FIG. 13, the clip 25 can be disengaged from the distal tip 24 and confined in the patient's body without ceasing to nip the affected region 11.

The same effects of the first configuration mentioned before can be obtained according to the present configuration. Since the retainer pipe 8 and the like for the clip 25 are omitted, the present configuration also has an effect that the device requires fewer components and can be manufactured at low cost. Further, manipulation can be simplified. Since the arm portions 25b of the clip 25 is fitted in the distal tip 24, moreover, dislocation between the unit of the clip 25 and the coil sheath 13 can be prevented when the forceps is raised, for example.

### (Exemplary embodiment of the present invention)

A clip manipulating device according to an embodiment of the present will be described with reference to FIGS. 14 and 15.

A clip manipulating device 26 for organic tissue according to the present embodiment differs from the third configuration in the configuration of a manipulating wire 27. The manipulating wire 27 of the present embodiment comprises a driving wire 27a, which is situated on the handling side, and a doubled thread 27c for use as a junction (coupling means) that is connected to the distal end of driving wire 27a by means of a joint 27b. A looped distal end portion of the doubled thread 27c serves as a junction that is anchored to a clip 7. That part (junction) of the thread 27c which is to be turned is passed through a proximal end portion (anchor portion) 7a of the clip 7 and anchored. When subjected to a given or heavier load, the turned wire portion of the thread 27c forms a weak point and snaps, whereupon the clip 7 is released. The joint 27b is situated behind an exposed portion 5b of a coil sheath 5. Thus, the driving wire 27a for use as the manipulating means couples the thread 27c as a flexible coupling member to the clip 7, and the joint 27b is located in a position at a suitable distance from the distal end of the coil sheath 5.

In order to bunch the thread elements on the doubled thread 27c or for other purposes, a very small rigid portion may be provided using an adhesive material or the like. A retainer pipe 28, which is similar to the one according to the first configuration, abuts against the distal end of the coil sheath 5. The present embodiment shares other features with the first configuration mentioned before.

The following is a description of the operation of the device according to the present embodiment. The present embodiment differs from the aforementioned third configuration in the process of disengagement of the clip unit 6. According to the present embodiment, the driving wire 27a is hauled to close the clip 7, whereupon the affected region 11 is grasped. If the driving wire 27a is further hauled in this grasping state, the thread 27c is broken by a tractive effort that exceeds a given value and disengaged from the proximal end portion 7a of the clip 7. The clip unit 6 comes off the manipulating wire 27. The present embodiment shares the effects with the third configuration mentioned before. Further, a side-viewing scope may be used to cope with a rigid joint, if any. If there is a rigid joint, moreover, the forceps raising device can be activated by means of the side-viewing scope to ensure smooth use.

The disengagement means according to the present embodiment that allows the wire portion to snap and release the clip 7 is also applicable to the first configuration mentioned before. Also available is an arrangement such that the wire portion of the stranded portion 19a according to the foregoing third configuration loosens so that the manipulating wire 19 is disengaged from the proximal end portion 7a of the clip 7.

### (Fifth Configuration, not forming part of the invention)

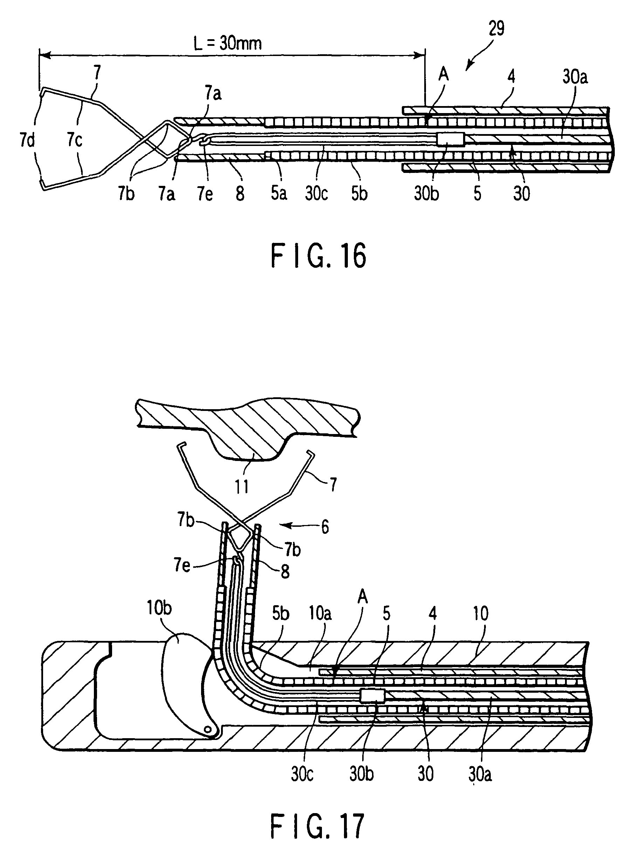

Clip manipulating devices according to a fifth configuration, which does not form part of the invention, will be described with reference to FIGS. 16 to 20.

Clip manipulating devices 29 and 31 according to the present configuration differ from the one according to the first configuration in an anchoring structure for a manipulating wire 30 and a clip 7.

First, the clip manipulating device 29 comprises a portion corresponding to a driving wire 30a of the manipulating wire 30 on the handling side and a doubled wire 30c that is connected to the distal end of the driving wire 30a by means of a joint 30b, as shown in FIG. 16. More specifically, in the doubled wire 30c, a turned distal end portion is hitched and connected to an anchor portion 7e of the clip 7, thereby forming a pliable coupling member portion. The doubled wire 30c is relatively long. Accordingly, the joint 30b is situated behind a position A at a distance L of 30 mm from the distal end of the clip 7, overreaching a range that covers at least a range corresponding to a movable range area that corresponds to the raising device of the endoscope. Thus, the joint 30b is always situated in a tube sheath 4 without projecting forward from the distal end of the tube sheath 4 or being exposed, as shown in FIGS. 16 and 17.

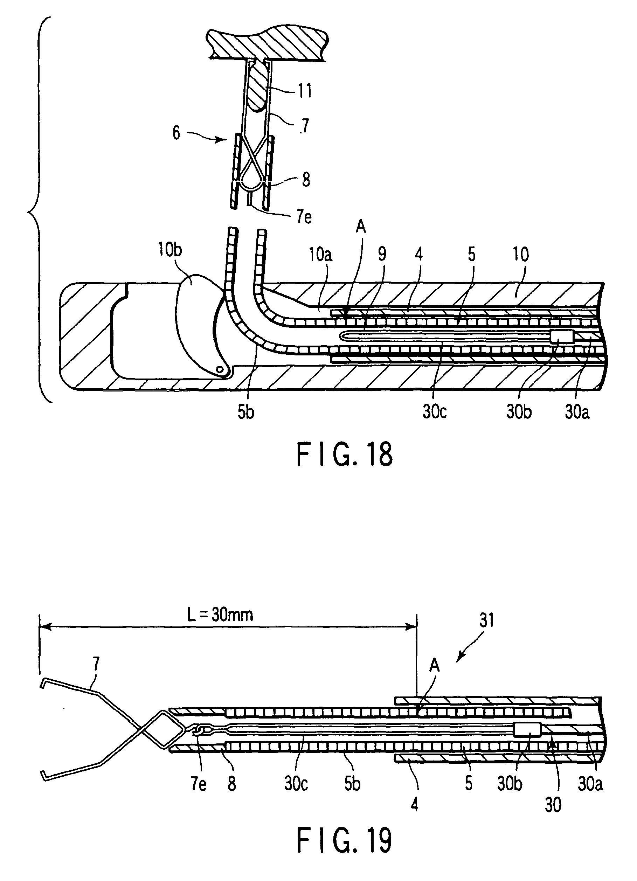

A clip manipulating device 31 is constructed in like manner, as shown in FIG. 19. The clip manipulating device 31 differs from the aforesaid one in that the basal part of the turned distal end portion of the doubled wire 30c is fastened so that the distal end side portion forms a looped portion. This looped portion is hitched to the anchor portion 7e of the clip 7.

The following is a description of the operation of the device according to the present configuration. The device is used in the steps of procedure shown in FIGS. 16 to 18. The present configuration shares this method of use with the first configuration. In this process, however, the joint 30b of the manipulating wire 30 can never get into the range from the distal end of the clip 7 to the position A at the backward distance of 30 mm from it. Further, it never passes through the range of the backward distance L of 30 mm from the distal end of the clip 7. Therefore, the joint 30b constitutes no hindrance to operation if it is rigid. In other words, there is no possibility of the rigid joint 30b passing through the coil sheath 5 that is bent by means of the forceps raising device 10b, so that the movement of the manipulating wire 30 cannot be hindered. Since a part (handling side portion) of the manipulating wire 30 can be formed into the thick driving wire 30a, moreover, the performance of the manipulating wire 30 to transmit force is improved.

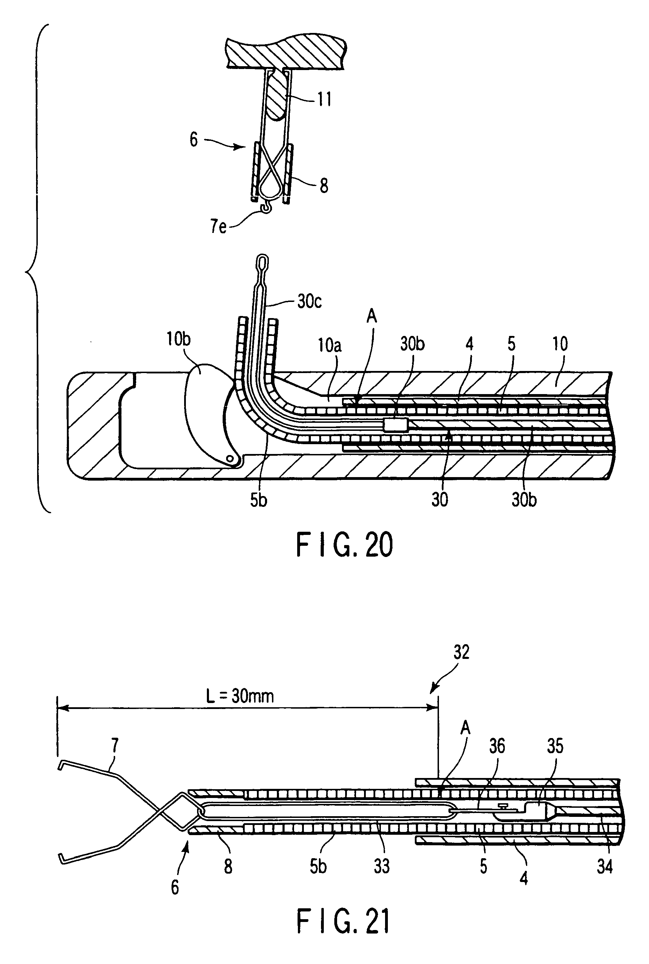

The clip manipulating device 31 shown in FIG. 19 is a modification of the foregoing clip manipulating device 29 according to the fifth configuration. The manipulating method for the disengagement of a clip unit 6 of the clip manipulating device 31 is different from the method for the clip manipulating device 29 according to the fifth configuration. After the affected region 11 is grasped by the clip 7, as shown in FIG. 20, the manipulating wire 30 is advanced to project the doubled wire 30c from the coil sheath 5. The distal end of the doubled wire 30c is disengaged from the anchor portion 7e of the clip 7, and the clip unit 6 is confined in the patient's body.

Also in this process of disengaging operation, the joint 30b of the manipulating wire 30 can never get into the range from the distal end of the clip 7 to the position A at the backward distance of 30 mm from it.

According to the present configuration, the joint 30b, a rigid portion, never passes through the range of the backward distance L of 30 mm from the distal end of the clip 7, so that the same effects of the first configuration can be obtained. Besides, a part of the manipulating wire 30 is formed into the thick driving wire 30a, so that the force transmission performance is improved further.

### (Sixth Configuration, not forming part the invention)

A clip manipulating device according to a sixth configuration, which does not form part of the invention, will be described with reference to FIGS. 21 and 22.

As shown in FIG. 21, a clip unit 6 of a clip manipulating device 32 according to the present configuration comprises a clip 7, a pliable looped wire 33 for use as a flexible connecting member hitched to the proximal end of the clip 7, and a retainer pipe 8. A hook 35 is provided on the distal end of a manipulating wire 34 for use as manipulating means. A coupling plate 36 is removably mounted on the hook 35, and the clip unit 6 is coupled to the coupling plate 36 of the manipulating wire 34 by means of the looped wire 33. The coupling plate 36 is formed having a J-shaped portion 36a as deformable anchor means on its distal end portion. The proximal end portion of the looped wire 33 is hitched to the anchoring J-shaped portion 36a, and the hook 35 is detachably coupled to the hook 35. As in the aforementioned case, the hook 35 and the coupling plate 36 are situated behind a position A at a backward distance L of 30 mm from the distal end of the clip 7, so that they cannot be projected and exposed through the distal end of the tube sheath 4.

The following is a description of the operation of the clip manipulating device 32 according to the present configuration. If the manipulating wire 34 is pulled further strongly after the affected region 11 is grasped by means of the clip 7, as shown in FIG. 22, the J-shaped portion 36a of the coupling plate 36 extends. Thereupon, the looped wire 33 is released from the anchored state, so that the clip unit 6 is disengaged. The clip unit 6 is confined in the patient's body. The present configuration shares the effects with the fifth configuration mentioned before.

The present invention is not limited to the embodiments described above, and may be applied to any other configurations.

In the present invention, the coupling means that is detachably coupled to the clip or the clip unit is not less pliable than enough to follow up the shape of the insertion tube. Even when the insertion tube is in the bent state, therefore, the coupling means can be smoothly moved in the insertion tube without hitching with ease.

In the present invention, the clip or the clip unit is provided with the anchor portion that is released from the manipulating means by means of the driving force of the given or higher value when it is driven by the manipulating means with the driving force. Accordingly, rigid regions on the manipulating means side can be reduced. Even when the insertion tube is in the bent state, therefore, the coupling means can be smoothly moved in the insertion tube without hitching with ease.

In the present invention, moreover, the coupling means, that is coupled to the clip or the clip unit and is released from the clip or the clip unit by means of the driving force of the given or higher value when it is driven with the driving force, is flexible. Even when the insertion tube is in the bent state, therefore, the coupling means can be smoothly moved in the insertion tube without hitching with ease.

In the present invention, the clip or the clip unit 6 is coupled with the flexible joint in the position at the suitable distance from the distal end of the insertion tube. Even when the insertion tube is in the bent state, therefore, any member of the coupling anchor means can be smoothly moved in the insertion tube without hitching with ease.

According to the present invention, the manipulating means that is coupled to the clip or the clip unit in the distal end of the insertion tube can be manipulated securely and quickly.

In the present invention, the clip or the clip unit is coupled to the manipulating means by means of the flexible connecting member that is coupled to the clip or the clip unit. The connecting member and the manipulating means are coupled by means of the anchor means that is located in the position at the suitable distance from the distal end of the insertion tube. The anchor portion releases the connecting member and the manipulating means from connection when the manipulating means is driven with the driving force of the given or higher value. Even when the insertion tube is in the bent state, therefore, any member of the coupling anchor means cannot hitch with ease.

## Claims

1. A clip manipulating device comprising:
a flexible insertion tube (5) capable of being inserted into a cavity of a living body;
a flexible wire (27) having pliability and movably passed through the insertion tube (5);
a junction provided on the distal end portion of the wire (27), detachably coupled with a clip (7) located at the distal end portion of the insertion tube (5) for effecting grasping operation and disengaging operation of the clip (7),
wherein the junction includes a looped flexible wire (27c), and is pliable enough to follow substantial bending deformation of the insertion tube (5), such that movement in the tube (5) is not hindered by the bending deformation;
**characterized in that** the loopeed flexible wire (27c) is coupled at its distal end with the clip (7), and at its proximal end to the flexible wire (27) at a joint (27b); and wherein said distal end of the looped flexible wire (27c) is adapted such as to break, when the wire (27) is hauled with a tractive effort great enough to release the clip (7).

2. A clip manipulating device according to claim 1, which comprises a flexible tube sheath (4) penetrated by the insertion tube (5) for advance and retreat, the tube sheath (4) being capable of storing the clip (7) located at the distal end portion of the insertion tube (5).

3. A clip manipulating device according to claim 2, wherein that part of the insertion tube (5) which is situated behind the clip (7) and exposed from the distal end of the tube sheath (4) when the clip (7) projects from the tube sheath (4) forms a curvedly raised portion.

4. A chip manipulating device according to claim 1, wherein the flexible insertion tube (5) forms a push member (17) for advancing the clip (7).

5. A clip manipulating device according to claim 1, wherein said insertion tube (5) has a distal end portion which may be curved and through which the looped flexible wire (27c) extends along the curved distal end portion, when the clip (7) is released, and
said joint (27b) is located apart from the cured distal end portion in the insertion tube upon a releasing of the clip.

6. A clip manipulating device according to claim 5, wherein said distal end portion of the insertion tube (5) is bent up to substantially 90 degrees by a forceps raising device (10b).

## Patentansprüche

1. Clip-Manipulationsgerät, aufweisend:
ein flexibles Einführrohr (5), das in einen Hohlraum eines lebenden Körpers eingeführt werden kann;
einen flexiblen Draht (27), der biegbar ist und beweglich durch das Einführrohr (5) verläuft;
eine Verbindungsstelle am distalen Endabschnitt des Drahtes (27), der mit einem Clip (7) am distalen Endabschnitt des Einführrohrs (5) lösbar gekoppelt ist, um eine Greif-und Freigabeoperation des Clips (7) zu bewirken,
wobei die Verbindungsstelle einen flexiblen Schlingendraht (27c) enthält und ausreichend biegbar ist, um einer starken Biegeverformung des Einführrohrs (5) zu folgen, so dass die Bewegung im Rohr (5) nicht durch die Biegeverformung behindert wird;
**dadurch gekennzeichnet, dass** der flexible Schlingendraht (27c) an seinem distalen Ende mit dem Clip (7) und an seinem proximalen Ende mit dem flexiblen Draht (27) an einer Verbindung (27b) gekoppelt ist; und
wobei das distale Ende des flexiblen Schlingendrahtes (27c) so ausgeführt ist, dass es reißt, wenn der Draht (27) mit einer Zugkraft gezogen wird, die groß genug ist, um den Clip (7) freizugeben.

2. Clip-Manipulationsgerät nach Anspruch 1, das einen flexiblen Rohrmantel (4) aufweist, der vom Einführrohr (5) zum Vorschub und Rückzug durchdrungen ist, wobei der Rohrmantel (4) den Clip (7) am distalen Endabschnitt des Einführrohrs (5) aufnehmen kann.

3. Clip-Manipulationsgerät nach Anspruch 2, bei dem der Teil des Einführrohrs (5), der sich hinter dem Clip (7) befindet und vom distalen Ende des Rohrmantels (4) freigelegt wird, wenn der Clip (7) aus dem Rohrmantel (4) herausragt, einen gekrümmt erhöhten Abschnitt bildet.

4. Clip-Manipulationsgerät nach Anspruch 1, bei dem das flexible Einführrohr (5) ein Schiebeelement (17) für den Vorschub des Clips (7) bildet.

5. Clip-Manipulationsgerät nach Anspruch 1, bei dem das Einführrohr (5) einen distalen Endabschnitt hat, der gekrümmt sein kann und durch den sich der flexible Schlingendraht (27c) entlang dem gekrümmten distalen Endabschnitt erstreckt, wenn der Clip (7) freigegeben wird, und
sich die Verbindung (27b) bei der Freigabe des Clips (7) entfernt vom gekrümmten distalen Endabschnitt im Einführrohr befindet.

6. Clip-Manipulationsgerät nach Anspruch 5, bei dem der distale Endabschnitt des Einführrohrs (5) von einem Hubgerät (10b) mit Zange bis zu im Wesentlichen 90° gekrümmt wird.

## Revendications

1. Dispositif de manipulation d'une pince, comprenant :
un tube d'insertion (5) flexible, capable d'être inséré dans une cavité d'un corps vivant ;
un fil flexible (27), présentant une aptitude au pliage et passé, de façon mobile, à travers le tube d'insertion (5) ;
une jonction, prévue sur la partie d'extrémité distale du fil (27), couplée de façon détachable à une pince (7) située à la partie d'extrémité distale du tube d'insertion (5), pour effectuer une opération de saisie et une opération de désengagement de la pince (7),
dans lequel la jonction comprend un fil (27c) flexible bouclé et est susceptible d'être pliée suffisamment pour suivre une déformation substantielle en flexion du tube d'insertion (5), telle que le déplacement dans le tube (5) ne soit pas entravé par la déformation en flexion ;
**caractérisé en ce que** le fil (27c) flexible bouclé est couplé, à son extrémité distale, à la pince (7) et, à son extrémité proximale, au fil flexible (27), au niveau d'un joint (27b) ; et dans lequel ladite extrémité distale du fil (27c) flexible bouclé est adaptée de manière à se rompre, lorsque le fil (27) est tiré avec un effort de traction suffisamment élevé, de manière à relâcher la pince (7).

2. Dispositif de manipulation d'une pince selon la revendication 1, comprenant une gaine de tube (4) flexible, pénétrée par le tube d'insertion (5) pour produire un avancement et une rétraction, la gaine de tube (4) étant capable de stocker la pince (7) située à la partie d'extrémité distale du tube d'insertion (5).

3. Dispositif de manipulation d'une pince selon la revendication 2, dans lequel la partie du tube d'insertion (5), située derrière la pince (7) et exposée à partir de l'extrémité distale de la gaine de tube (4) lorsque la pince (7) fait saillie de la gaine de tube (4), forme une partie levée de façon incurvée.

4. Dispositif de manipulation d'une pince selon la revendication 1, dans lequel le tube d'insertion (5) flexible forme un organe de poussée (17) servant à faire avancer la pince (7).

5. Dispositif de manipulation d'une pince selon la revendication 1, dans lequel ledit tube d'insertion (5) comprend une partie d'extrémité distale, pouvant être incurvée et à travers laquelle le fil (27c) flexible bouclé s'étend le long de la partie d'extrémité distale incurvée, lorsque la pince (7) est relâchée, et
ledit joint (27b) est situé à distance de la partie d'extrémité distale incurvée dans le tube d'insertion, lors d'un relâchement de la pince.

6. Dispositif de manipulation d'une pince selon la revendication 5, dans lequel ladite partie d'extrémité distale du tube d'insertion (5) est coudée vers le haut, sensiblement à 90 degrés, par un dispositif de levée de forceps (10b).
